Europäisches Patentamt

European Patent Office

Office européen des brevets

(11) Publication number: **0 575 906 A2**

# EUROPEAN PATENT APPLICATION

(21) Application number: **93109764.6**

(22) Date of filing: **18.06.93**

(83) Declaration under Rule 28(4) EPC (expert solution)

(51) Int. Cl.5: **G01N 33/573, //C12Q1/34**

The applicant has filed a statement in accordance with Rule 28 (4) EPC (issue of a sample only to an expert). Accession number(s) of the deposit(s): FERM 3858, 3859 and 3860.

(30) Priority: **19.06.92 JP 186194/92**

(43) Date of publication of application:
**29.12.93 Bulletin 93/52**

(84) Designated Contracting States:
**AT BE CH DE DK ES FR GB GR IE IT LI LU MC NL PT SE**

(71) Applicant: **SHIONOGI SEIYAKU KABUSHIKI KAISHA trading under the name of SHIONOGI & CO. LTD.**
**1-8, Doshomachi 3-chome**
**Chuo-ku**
**Osaka 541(JP)**

(72) Inventor: **Numata, Yoshito**
**3-1-27-308, Yamamoto-cho kita**
**Yao-shi, Osaka(JP)**
Inventor: **Kosugi, Yoko**
**4-4-3, Takakuradai**
**Sakai-shi, Osaka(JP)**
Inventor: **Shibata, Kazunori**
**3-2-2-401, Shinkitano,**
**Yodogawa-ku**
**Osaka-shi, Osaka(JP)**

(74) Representative: **VOSSIUS & PARTNER**
**Postfach 86 07 67**
**D-81634 München (DE)**

(54) **Sandwich immunoassay of beta-N-acetylglucosaminidase and monoclonal antibody used therein.**

(57) The present invention relates to a sandwich immunoassay of NAG, which comprises sandwiching NAG between an immobilized anti-NAG monoclonal antibody and a labeled anti-NAG monoclonal antibody.

This sandwich immunoassay is useful for diagnosis of renal disease, hepatitis, leukemia, and the like because it makes possible to directly and specifically detect NAG isozymes B and I in urine and blood.

EP 0 575 906 A2

Rank Xerox (UK) Business Services
(3.10/3.6/3.0.2)

The present invention relates to a sandwich immunoassay of $\beta$-N-acetylglucosaminidase (NAG) and a monoclonal antibody which recognizes NAG and is useful for said sandwich immunoassay. More particularly, it relates to a sandwich immunoassay of NAG isozyme B (and I) and a monoclonal antibody which recognizes NAG isozyme B (and I) and is useful for said sandwich immunoassay.

$\beta$-N-acetylglucosaminidase (NAG or $\beta$-hexosaminidase, M.W. :c.a. 120,000) is a widely distributed lysosomal enzyme, located predominantly in the renal proximal tubules. Increased NAG enzymatic activity in urine has been found to be associated with various kidney injuries. An assay for urinary NAG activity developed by Noto et al. (Noto, A., et al., Clin. Chem. 29, 1713, 1983) has been used for diagnosis and observation of progress of rejection after renal transplantation, drug nephrotoxicity and acute renal insufficiency because the assay is simple, sensitive, and reproducible.

NAG can be separated into some isozymes. In urine of a normal healthy human, NAG isozyme A accounts for 80 - 90 % and NAG isozyme B does 10 - 20 %. NAG isozyme A is composed of two different subunits $\alpha$ and $\beta$, whereas NAG isozymes B and I consist of two $\beta$-subunits. These isozymes differ in isoelectric point, substrate specificity, thermal stability etc.

There have been many reports describing the clinical significance of NAG isozymes, especially the rise of the ratio of NAG isozymes B and I in urine of patients with renal tubular injury caused by aminoglycoside antibiotics (Gibey, R., et al., Clin. Ohim. Acta. 137, 1, 1984), pyelonephritis (upper urinary-tract infection) (Vigono, A., et al., Clin. Chim. Acta. 130, 297, 1983), renal transplantation (Yuen, C-T., Clin. Chim. Acta. 164, 339, 1987), and the like. Therefore, a specific assay for NAG isozymes B and I makes it possible to diagnose renal damage more sensitively than an assay for total NAG.

Up to now, the separation of NAG isozymes has been performed by ion-exchange chromatography (Ellis, B. G. et al., Clin. Chim. Acta. 64, 195, 1975), electrophoresis (Pitkanen, E. et al., Enzyme 28, 14, 1982), heat treatment (Oberketter, L. V. et al., Clin. Chim. Acta. 94, 281, 1979), or the like. However, these methods are not adopted to routine clinical work because of their defect such as low sensitivity, complicated operation, and bad precision.

Recently, an enzyme immunoassay (EIA) for NAG isozyme A or B using monoclonal antibodies was reported (A. ISAKSSON, et al., Scand. J. Clin. Lab. Invest. 49, 597-603, 1989). This assay was not a sandwich assay wherein NAG is sandwiched between two antibodies, but an antigen capture assays wherein NAG is immunologically bound to an immobilized anti-NAG monoclonal antibody and an enzymatic activity of the bound NAG is measured. In this assay, however, NAG isozymes could not be quantitatively detected because the used anti-NAG monoclonal antibody inhibited the enzymatic activity of NAG.

Thus, the technical problem underlying the present invention was to provide highly sensitive and quantitative assays for NAG isozymes B and I. The solution to this technical problem was achieved by prviding a sandwich immunoassay of NAG, which comprises sandwiching NAG between an immobilized anti-NAG monoclonal antibody and a labeled anti-NAG monoclonal antibody. The sandwich immunoassay of this invention makes it possible to quantitatively detect NAG because the detection is based on the label's activity of the labeled monoclonal antibody but not on the enzymatic activity of NAG. Furthermore, as this sandwich immunoassay is performed by sandwiching NAG between two different monoclonal antibodies against NAG isozymes B, NAG isozyme B is detected in this sandwich immunoassay more selectively than in the method using only one anti-NAG monoclonal antibody.

Regarding to the clinical significance of NAG isozymes, it has been reported that in pathological urine, the excretion of NAG isozymes B and I was elevated by renal injury due to aminoglycoside antibiotics, interstitial damage such as upper urinary-tract infection, renal transplantation, and the like. The elevation of NAG isozyme I has also been reported in blood of patients with hepatitis, leukemia, etc. However, the usual separation methods of NAG isozymes, that is chromatography, electrophoresis, and heat treatment, are not easily adopted to routine clinical work because of their defects such as low sensitivity, complicated operation, and bad precision. The sandwich immunoassay of this invention makes it possible to directly and specifically detect NAG isozymes B and I in urine and blood as a sensitive marker of renal disease, hepatitis, leukemia, and the like.

The figures show:

Fig. 1

(A) Titer of the present monoclonal antibody Hex 31 at the various concentration to NAG isozyme A (-○-) and B (-●-).
(B) Titer of the present monoclonal antibody Hex 32 at the various concentration to NAG isozyme A (-○-) and B (-●-).

Fig .2

(A) Enzymatic activity of NAG isozyme A (-○-) and B (-●-) after binding to the present monoclonal antibody Hex 31 with various concentration.
(B) Enzymatic activity of NAG isozyme A (-○-) and B (-●-) after binding to the present monoclonal antibody Hex 32 with various concentration.

Fig. 3

Standard calibration curve of the present sandwich RIA using NAG isozyme B as a standard antigen.

Fig. 4

(A) Ion-exchange chromatography of partially-purified NAG isozyme A from human placenta. Fractions were subjected to the present sandwich RIA (-●-) and enzyme assay (-○-).
(B) Ion-exchange chromatography of partially-purified NAG isozyme B from human placenta. Fractions were subjected to the present sandwich RIA (-●-) and enzyme assay (-○-).

Fig. 5

(A) & (B) Ion-exchange chromatography of NAG isozymes in urine samples. Fractions were subjected to the present sandwich RIA (-●-) and enzyme assay (-○-).

Fig. 6

Standard calibration curve of the present sandwich EIA using NAG isozymes B (-●-) and A (-○-) as a standard antigen.

Fig. 7

Dilution curves of three urinary samples having different concentration.

The sandwich immunoassay of this invention comprises the following steps:

(1) reacting an immobilized anti-NAG monoclonal antibody and a labeled anti-NAG monoclonal antibody with a sample containing NAG to form a complex of immobilized antibody-NAG-labeled antibody and
(2) detecting label's radioactivity or enzymatic activity of said labeled monoclonal antibody which is contained or is not contained in the complex.

In the above process (1), the reaction of said immobilized monoclonal antibody and said labeled monoclonal antibody with a sample containing NAG may be carried out either simultaneously or separately.

The present sandwich immunoassay may further comprise the following steps:

(1) immobilizing an anti-NAG monoclonal antibody on a carrier to give a immobilized monoclonal antibody and
(2) labeling an anti-NAG monoclonal antibody which recognizes a different site from that recognized by the immobilized monoclonal antibody with a radioisotope or an enzyme to give a labeled monoclonal antibody.

Using anti-NAG isozyme B monoclonal antibodies as anti-NAG monoclonal antibody in this sandwich immunoassay, NAG isozyme B can be quantitatively and specifically detected. The anti-NAG isozyme B monoclonal antibodies include monoclonal antibody Hex 31 and Hex 32 which are produced by Hybridoma Hex 31 and Hex 32, respectively. In this invention, Hex 31 as an immobilized antibody and Hex 32 as a labeled antibody are preferably used, of cause, inverted combination of this is applicable. Further, it is applicable that Hex 31 or Hex 32 may be combined with another monoclonal or polyclonal antibody which recognizes different site of NAG isozyme B from that recognized by Hex 31 or Hex 32.

It is clear from the Example described later that in this sandwich immunoassay, NAG isozyme I, if present, is also detected as well as B. Therefore, the present sandwich immunoassay further likes it possible to measure the total amount of NAG isozymes B and I in the case that they co-exist.

This invention further provides a monoclonal antibody recognizing NAG, which is useful for the sandwich immunoassay of this invention. The present monoclonal antibody preferably includes anti-NAG isozyme B monoclonal antibody such as monoclonal antibody Hex 31 produced by Hybridoma Hex 31 and monoclonal antibody Hex 32 produced by Hybridoma Hex 32. Hybridoma Hex 31 and Hybridoma Hex 32 have been deposited with Fermentation Research Institute at 1-3, Higashi 1-chome, Tsukuba-shi, Ibaraki-ken, 305, Japan since May 20, 1992 under Budapest Treaty as "Mouse hybridoma Hex 31" with accession No. FERM BP-3858 and "Mouse hybridoma Hex 32" with accession No. FERM BP-3859, respectively.

Considering that both NAG isozymes B and I can be detected by the present sandwich immunoassay and that NAG isozymes B and I consist of two β-subunits and they structurally differ in only sugar chain, it is concluded that the monoclonal antibody of this invention recognizes both NAG isozymes B and I and the epitope does not include their sugar chain. Further, the present monoclonal antibody does not inhibit enzymatic activity of NAG as described in the following Example. Therefore, the present antibody recognizes the different site of NAG from that recognized by ISSAKSON's monoclonal antibody.

A method of preparing the present monoclonal antibody and the sandwich immunoassay of this invention are simply described below.

### 1. Preparation of hybridoma producing anti-NAG monoclonal antibody and Production of the antibody.

#### (1) Immunization & Cell fusion

NAG, preferably NAG isozyme B, is emulsified in an appropriate adjuvant such as Freund's complete adjuvant. The emulsion is intramuscularly inoculated into mice repeatedly at intervals of a few months. The NAG isozyme B as an immunogen may be either crude or partially purified, or purified NAG isozyme B, which is purified using anti-NAG isozyme B monoclonal antibody, preferably monoclonal antibody Hex 33 produced by Hybridoma Hex 33, can also be used. Hybridoma Hex 33 has been deposited with Fermentation Research Institute at 1-3, Higashi 1-chome, Tsukuba-shi, Ibaraki-ken, 305, Japan as " Mouse hybridoma Hex 33" with accession No. FERM BP-3860 since May 20, 1992 under Budapest Treaty.

The spleens of the mice are taken 3 to 5 days after the last immunization to be used as antibody-producing cells. Myeloma cells having a suitable marker such as 8-azaguanine resistance are used as parent cells which are fused with the anitbody-producing cells to prepare hybridomas. As a medium for the preparation of hybridomas, an ordinary medium such as Eagle's MEM, Dulbecco's modified medium, RPMI-1640, or the like may be used.

At first, myeloma cells as parent cells and spleen cells are prepared at a ratio of c.a. 1 : 5. As a fusing agent, polyethylene glycol (PEG) is used at a concentration of 50 % for the efficient fusion. Resulting cells are selected by the HAT method. Screening of obtained hybridomas is performed by the conventional method such as an immunoassay using culture supernatant of the hybridomas to give a clone of hybridoma producing objective immunoglobulin. The obtained antibody-producing hybridoma is cloned a few times by the known method such as limiting dilution method.

#### (2) Production of monoclonal antibody

In order to produce the monoclonal antibody of this invention, the hybridoma obtained above is cultured in vitro or in vivo. In the case of in vitro culture, the hybridoma is cultured in the above-mentioned ordinary medium supplemented with FCS for 3 - 5 days to recover a monoclonal antibody from the culture supernatant. In the case of in vivo culture, the hybridoma is implanted to the abdominal cavity of a mammal, and after 1 - 3 weeks the ascitic fluid is collected to recover a monoclonal antibody therefrom. In the case of the in vivo culture, a much larger quantity of the monoclonal antibody can efficiently be obtained than that in the case of the in vitro culture, and the in vivo culture is therefore preferable.

The monoclonal antibody obtained from the supernatant or ascitic fluids can be purified by the conventional method such as ammonium sulfate-fractionation, Protein G-Sepharose column chromatography, or their combinations.

### 2. Sandwich immunoassay of NAG

#### (1) Preparation of immobilized antibody

As a solid phase on which an antibody is immobilized, a commercially available ordinary carriers for the antigen-antibody reaction such as bead, ball, tube, and plate made of glass or synthesized resin can be used. The antibody obtained in above item 1. (2) is adsorbed on the carrier by allowing it to stand at a room temperature overnight in phosphate buffer of pH 6 - 10, preferably around neutral. The antibody-adsorbing carrier is stored with cooling in the presence of a preservative such as sodium azide. Either monoclonal or polyclonal antibody can be immobilized on the carrier by the above method.

(2) Preparation of labeled antibody

As a substance to label an antibody, all substances usually used for an immunoassay such as radioisotopes, enzymes, and fluorescent substances can be used. In this invention, radioisotopes and enzymes are preferably used. In the case of radioisotopes, the antibody is labeled with a radioisotope such as $^{125}$I by the known method such as Chloramine T method. In the case of enzymes, the antibody is labeled with an enzyme such as horseradish peroxidase, $\beta$-D-galactosidase, or alkaliphosphatase by the known method such as the maleimido method and the hingi method.

(3) Assay of NAG

Using the immobilized antibody and the labeled antibody prepared in above items (1) and (2), respectively, a sandwich immunoassay of NAG is carried out. The assay is preferably a two-step sandwich method which comprises the following steps: (1) reacting the immobilized antibody with a NAG-containing sample such as a NAG standard antigen solution, urine, and blood at 4 - 40 °C, preferably 20 - 30 °C for 0.5 - 24 hr, preferably 1 - 3 hr to form a complex of immobilized antibody - NAG, (2) reacting the complex of immobilized antibody - NAG with the labeled antibody at 4 - 40 °C, preferably 20 - 30 °C for 0.5 - 24 hr, preferably 1 - 3 hr to form a complex of immobilized antibody - NAG - labeled antibody, and (3) detecting label's activity of the labeled antibody which is bound or is not bound to said complex. Of cause, one-step method in which the process (1) and (2) are simultaneously carried out is applicable. Furthermore, it is applicable that after reacting the labeled antibody with the sample to form a complex of labeled antibody - NAG, the immobilized antibody is reacted with the complex of labeled antibody - NAG to form a complex of labeled antibody - NAG - immobilized antibody.

The detection of label's activity can be performed by conventional methods in accordance with the label substance. In case of radioisotopes, it can be detected using an appropriate instrument such as a $\gamma$-counter. In case of enzymes, it can be detected by measuring absorbence, fluorescence intensity, or luminescence intensity after reacting the enzyme with a appropriate substrate.

The present invention further provides a kit for measurement of NAG, which comprises an immobilized anti-NAG monoclonal antibody and a labeled anti-NAG monoclonal antibody, wherein NAG can be sandwiched between said immobilized monoclonal antibody and said labeled monoclonal antibody.

Using anti-NAG isozyme B monoclonal antibodies as anti-NAG monoclonal antibody in the present kit, NAG isozyme B can be quantitatively and specifically detected. As the anti-NAG isozyme B monoclonal antibodies, monoclonal antibody Hex 31 and Hex 32, which are produced by Hybridoma Hex 31 and Hex 32, respectively, are illustrated as described before. In this kit, Hex 31 as an immobilized antibody and Hex 32 as a labeled antibody are preferably used, of cause, inverted combination of this is applicable. Further, it is applicable that Hex 31 or Hex 32 may be combined with another monoclonal or polyclonal antibody which recognizes different site of NAG isozyme B from that recognized by Hex 31 or Hex 32.

As the monoclonal antibodies used for this kit can recognize both NAG isozyme B and I when they co-exist as mentioned above, the total amount of NAG isozymes B and I can be measured by the kit of this invention.

This invention is illustrated by the following Example

1. Sandwich RIA of NAG

(1) Preparation of hybridoma producing anti-NAG monoclonal antibody and Production of the antibody.

(a) Immunization

Using partially purified $\beta$-N-acetylglucosaminidase isozyme B from human placenta (Sigma Chemical Co.) as an immunogen, Balb/c mice (4 weeks) were immunized according to the following procedure.

The enzyme was centrifuged at 1,200 rpm for 5 min because it was on the market as a suspension in 2.4 M ammonium sulfate. The resulting precipitate was dissolved in physiological saline and mixed with Freund's complete adjuvant. At the first immunization, the enzyme (10$\mu$ g/mouse) was intraperitoneally injected into mice. Subsequently, the mice received three intramuscularly injections of the same amount of the enzyme at 2-month intervals.

(b) Cell fusion

Four days after the last injection, titer of antiserum collected from immunized mice was measured according to the procedure mentioned in the following item (c). Spleens were excised from the mice which have significant titer, and the obtained splenic cells were allowed to stand in 0.17 M ammonium chloride at freezing point for 5 min to destroy erythrocytes. The remaining cells were suspended in RPMI 1640 medium to give splenic lymphocytes to be used in cell fusion. A mixture of myeloma cells ($6.3 \times 10^7$ cells) resistant to 8-azaguanine suspended in RPMI 1640 medium and the splenic lymphocytes (X63.653, $3.5 \times 10^8$ cells) were centrifuged and the resulting supernatant was removed. To the obtained cell precipitate was added 0.8 ml of 50% polyethylene glycol (M.W. 4, 000, Merck) in 1 min with stirring by a top of a pipet, and then the mixture was stirred for 1.5 min. To the mixture was added 2 ml of RPMI 1640 medium in 2 min and then 2 ml of that in 1 min. Furthermore, 18 ml of RPMI 1640 medium was added dropwise with gentle agitation.

After centrifugation, the resulting supernatant was removed. The obtained cell precipitate was suspended in 100 ml of HAT medium (20 % FCS - RPMI 1640 medium supplemented with $1 \times 10^{-4}$ M hypoxanthine, $4 \times 10^{-7}$ M aminopterin, and $1.6 \times 10^{-5}$ M thymidine) and the suspension (0.1 ml/well) was seeded in twelve 96-well tissue culture plates (Falcon). To each well was added 50 $\mu$l and 100 $\mu$l of HAT medium after 2 days and 7 days, respectively. After 10 days, the culture supernatant was collected and subjected to screening by the method described in the following item (c). The obtained hybridomas producing anti-NAG antibodies were cloned three times by the limiting dilution method to give hybridoma Hex 31, Hex 32, and Hex 33.

(c) Measurement of titer of antibody

Microtiter plate (Dynatech Lab. Inc.) was coated with rabbit anti-mouse IgG (10 $\mu$g/100 $\mu$l/well) in PBS (10 mM phosphate buffer containing 0.15 M sodium chloride, pH 7.4) and left at 4 °C overnight. The wells were washed once with PBS containing 1 % bovine serum albumin (BSA) and incubated with the same buffer (200 $\mu$l/well) at 37 °C for 1 hr. After the wells were washed three times with PBS containing 0.05 % Tween 20, 100 $\mu$l of the antiserum or the culture supernatant containing hybridoma was added to the wells and incubated at room temperature for 2 hr. After washing three times, partially purified NAG isozyme A or B from human placenta (Sigma Chemical Co.) (0.5 mU/100 $\mu$l) in PBS containing 0.1 % BSA was added and incubated at room temperature for 2 hr. After washing three times, 1 mM 4-methylumbelliferyl-2-acetamide-2-deoxy-$\beta$-D-glucopyranoside in 50mM citrate-phosphate buffer, pH 4.9 (100 $\mu$l) was added and incubated at 37 °C for 2 hr (enzyme reaction). The enzyme reaction was stopped by addition of 0.5 M glycine-NaOH, pH 10.5 (100 $\mu$l). The fluorescence intensity of 4-methylumbelliferone released in each well was measured at an excitation wavelength of 365 nm and an emission wavelength of 450 nm, using a microplate-reader (MTP-32, Corona Electric Co, Ltd.).

(d) Preparation of ascitic fluid

Hybridoma Hex 31 or Hex 32 ($2 \times 10^6$ cells) was injected intraperitoneally into mice to which 0.2 ml of pristan had been injected intraperitoneally 21 days before. The ascitic fluid was suitably collected after 1 - 3 weeks, centrifuged to remove cells therein, and preserved at -80 °C.

(e) Purification of monoclonal antibody and Confirmation of titer of the antibody

The ascitic fluid (20 ml) preserved at -80 °C was molten, diluted twice with the same volume of PBS, and then loaded onto a Protein G Sepharose 4 Fast Flow column (1.5 13 cm, Pharmacia) equilibrated with 20 mM phosphate buffer, pH 7.0 (abbreviated as Buffer A, hereinafter). After washing the column with 60 ml of Buffer A, mouse IgG was eluted with 10 mM glycin-HCl buffer, pH 2.7 (60ml). The eluate was neutralized with 1M Tris and then treated with 50 % ammonium sulfate at 0 °C. After centrifugation at 6,000 rpm for 30 min, the resulting precipitate was dissolved in 10 ml of PBS and dialyzed at 4 °C overnight against PBS to give purified antibody.

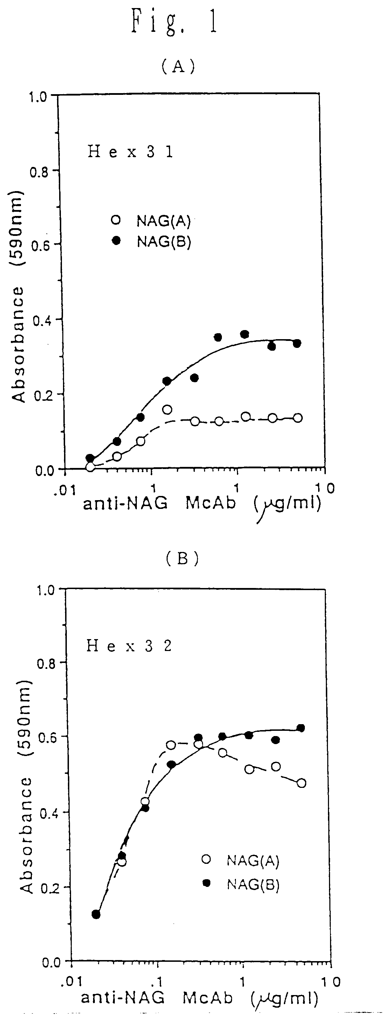

Confirmation of titer of the purified antibody was carried out according to the method described in above item (c), provided that the enzyme reaction was performed for 30 min using sodio-m-cresolsulfonphtaleinyl-H-acetyl-$\beta$-D-glucosaminide as a substrate and the absorbance was determined at 590 nm. As a result, the antibodies had significant titer even at a concentration of 0.1 $\mu$g/ml as shown in Fig. 1. Both monoclonal antibody Hex 31 and Hex 32, which were produced by Hybridoma Hex 31 and Hex 32, respectively, reacted very well with NAG isozyme B (-●-) and slight cross-reactivity to NAG isozyme A was observed (-O-).

### (f) Typing of antibody

Isotype determination of the immunogloblin produced by the hybridoma was carried out by the enzyme immunoassay method using an Isotyping Kit (Phar Minigen). As a result, both anti-human NAG monoclonal antibodies Hex 31 and Hex 32 belong to $IgG_1 . \chi$.

### (g) Inhibition of enzymatic activity of NAG by antibody

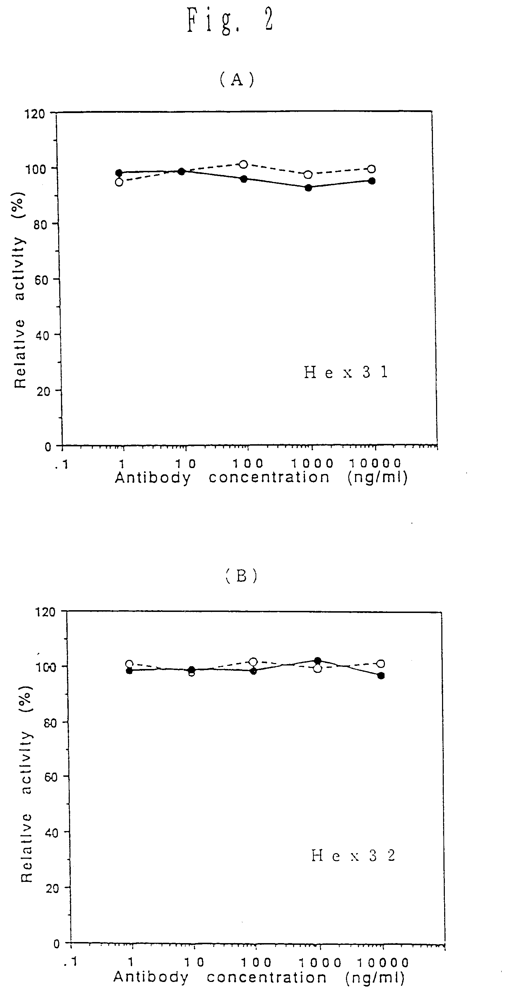

Using 0.1 % BSA-containing PBS, 110U/L NAG isozyme A or B and 0.1 -10,000 ng/ml anti-human NAG monoclonal antibody Hex 31 or Hex 32 are prepared, and they are reacted at room temperature for 2 hr. After the reaction, the enzymatic activity of NAG was measured using NAG Test Shionogi (Shionogi & Co. Ltd.). As shown in Fig. 2, the enzymatic activities of NAG isozyme A (-O-) and B (-●-) were not inhibited at all by even excess of antibodies.

### (2) Sandwich immunoassay of NAG

### (a) Radioisotopic labelling

In 0.5 M PB, pH 7.5 (50 $\mu$l, abbreviated as Buffer B, hereinafter) was dissolved 25 $\mu$g of purified antibody Hex 32, to which 0.5 mCi Na $^{125}$I (5 $\mu$l/25 $\mu$l) and Chloramine T (50 $\mu$g/25 $\mu$l) in Buffer B were added, followed by reaction at room temperature for 15 sec. The reaction was stopped by addition of sodium disulfite (100 $\mu$g/25 $\mu$l) in Buffer B and potassium iodide (2.5 mg/20 $\mu$l) in water. The reaction solution was loaded onto PD-10 column (Pharmacia) equilibrated with PBS containing 0.5 % BSA and 0.1 % sodium azide to collect fractions including labeled antibody.

### (b) Sandwich RIA

Microtiter plate (Dynatech) were coated with purified antibody Hex 31 (1 $\mu$g/100 $\mu$l/well) in PBS and left at 4 °C overnight. The wells were washed once with PBS containing 1 % BSA and incubated with the same buffer (200 $\mu$l/well) at 37 °C for 1 hr. The wells were washed three times with PBS containing 0.05 % Tween 20, and then 75 $\mu$l of 0.2 M citrate-phosphate buffer (pH 5.0) containing 0.1 % BSA and 0.02 % sodium azide and 25 $\mu$l of standard antigen solution or urinary sample were added to the wells, followed by incubation at room temperature for 2 hr (1st reaction). After washing the wells three times, $^{125}$I-labeled Hex 32 (c.a. $10^5$ cpm/10 ng/100 $\mu$l) in 0.2 M citrate-phosphate buffer (pH 7.0) containing 0.1 % BSA and 0.02 % sodium azide was added, followed by allowing to stand at room temperature for 2 hr (2nd reaction). After the reaction, the wells were washed three times and cut out, and the radioactivity of each well was determined with $\gamma$ -counter (ARC-600, Aloca).

As the standard antigen solution, partially purified human placental NAG isozyme A or B (Sigma Chemical Co.) (0.5 - 20 U/L as enzymatic activity) was used.

Optimal pH for the 1st and 2nd reactions in the above-mentioned sandwich RIA had been determined using 0.2M citrate-phosphate buffer (pH 4.5 - 8.0) containing 0.1 % BSA and 0.02 % sodium azide according to the method described below.

At first, the pH of the 2nd reaction was fixed at 7.0 and the pH of the 1st reaction was varied. As a result, the cross-reactivity to NAG isozyme A amounted to 33 % at pH 7.0, but it reduced as the pH of 1st reaction fell, so that it amounted to 15 % at pH 5.0 (it was appeared that the 15 % cross-reactivity was not due to NAG isozyme A, but NAG isozyme I contaminating in the standard antigen solution of NAG isozyme A as described below). At pH 4.5, no reactivity to both NAG isozyme A and B was observed. Next, the pH of 1st reaction was fixed at pH 5.0 and the pH of the 2nd reaction was varied. As a result, there was no change in the cross-reactivity to NAG isozyme A (13-17 %). The effects of the pHs in the 1st and 2nd reaction upon the reactivity to NAG isozymes were appeared to be due to the characteristic of the immobilized antibody Hex 31 and the labeled antibody Hex 32, respectively. From the above results, pH 5.0 for the 1st reaction was selected to decrease the cross-reactivity to NAG isozyme A and pH 7.0 for the 2nd reaction to increase the sensitivity.

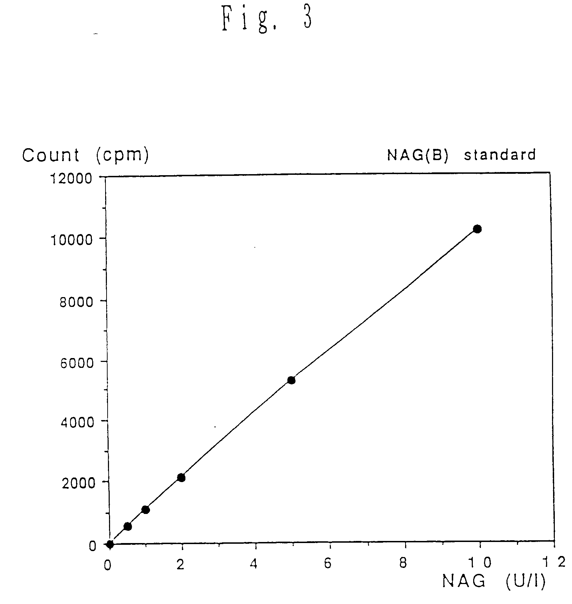

A standard calibration curve of the present sandwich RIA using NAG isozyme B as a standard antigen was shown in Fig. 3. It was excellently linear over the range from 0 to 10 U/L.

The reproducibility of the present RIA was shown in Table 1. It was estimated using the standard antigen solution and eight urine samples. The coefficients of variation (C.V.) of the within-assay series were less than 10 % (n = 5).

(3) Analysis of NAG isozymes by ion-exchange chromatography

The specificity of the present sandwich RIA to NAG isozymes was examined by ion-exchange chromatography method. The separation of NAG isozymes was performed by FPLC system (Pharmacia using Mono Q column (0.5 × 5.0 cm). The elution was done with 10 mM phosphate buffer (pH 6.0) at a flow rate of 1 ml/min according to the liner concentration gradient method. The amount of NAG in the resulting fractions was measured by both the present RIA and enzyme assay using NAG Test Shionogi (Shionogi & Co. Ltd.) in which sodio-m-cresolsulfonphtaleinyl-N-acetyl-$\beta$-D-glucosaminide was used as a substrate.

As a sample for the analysis of NAG isozymes, partially purified NAG isozyme A or B from human placenta (Sigma Chemical Co.) and urinary samples which were concentrated 5 to 10-fold were used. Those samples were dialyzed at 4 ° C overnight against 10 mM phosphate buffer (pH 6.0) and then injected into column after adding 0.1 % BSA as a carrier protein.

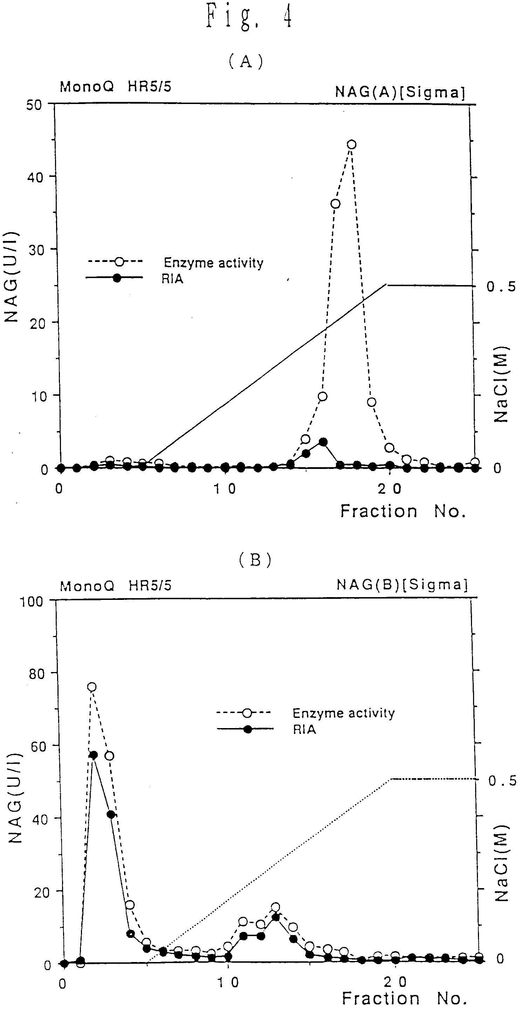

As shown in Fig. 4 (A) (the elution pattern of NAG isozyme A from human placenta), the peak (fraction No. 16) of the RIA (-O-) appeared faster than that of the enzyme assay (-●-). The peak was probably due to NAG isozyme I contaminating in partially purified NAG isozyme A as an antigen because (1) as two anti-NAG isozyme B monoclonal antibodies were used in this RIA, the selectivity of the RIA to NAG isozyme B was very high and the possibility of the cross-reactivity to isozyme A having quite different subunits from isozyme B is negligible, (2) as NAG isozyme I is composed of two $\beta$-subunits as well as isozyme B and differs from NAG isozyme B in only sugar chain, there is a great possibility that the monoclonal antibodies used in this RIA recognize NAG isozyme I as well as isozyme B.

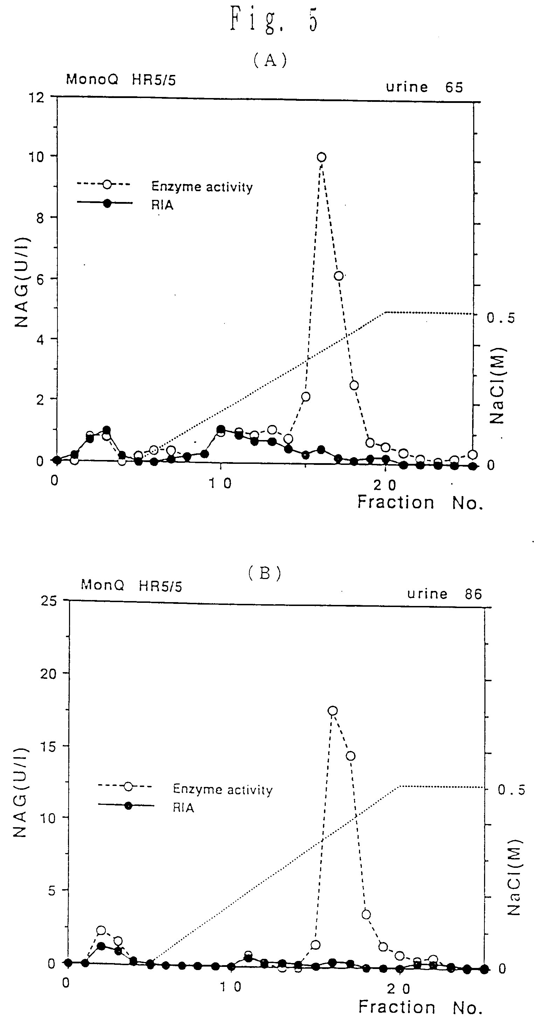

As shown in Fig. 4 (B), the elution pattern of NAG isozyme B from human placental by the RIA was identical with that by the enzyme assay, in which patterns appeared peaks (fraction No. 11 - 13) due to contamination of isozyme I. In case of the elution patterns of two urinary samples (Fig. 5), the peaks due to NAG isozyme A (fraction No. 16), B (fraction No. 2 - 4), and I (fraction No. 10 - 11) appeared in the enzyme assay, but there is no peak due to isozyme A in the present RIA. These results indicated that the amount of NAG detected by the present RIA reflected those of isozymes B and I.

2. Sandwich EIA of NAG

(1) Purification of NAG isozyme B from human placenta

Purification of NAG isozyme B used as an antigen comprised the following steps: 1) ammonium sulfate-fractionation, 2) adsorption of glycoprotein by Con A-Sepharose (Pharmacia) chromatography, 3) separation of NAG isozyme B from NAG isozymes by antibody-column chromatography with use of monoclonal antibody Hex 33 produced by Hybridoma Hex 33 obtained in above item 1. (1) (b) and by Mono Q column (Pharmacia) chromatography, and 4) purification by Sephacryl S-200 (Pharmacia). The anti-NAG antibody-column was prepared according to Schneider's method (J. Biol. Chem. 257, 10766-10769, 1982). The enzymatic activity of NAG was measured by NAG Test Shionogi (Shionogi & Co., Ltd.) using sodio-m-cresolsulfonphtaleinyl-N-acetyl-$\beta$-D-glucosaminide as a substrate. The detailed procedure was described below (all operations in the procedure were carried out at 4 ° C).

Twelve human placenta without funiculus umbilicals and capsula were washed a few times with distilled water to remove erythrocyte, cut to pieces, and then homogenized with 25 mM sodium phosphate buffer, pH 6.0 (twice volume). The obtained homogenate was centrifuged at 10,000 ×g for 20 min. The resulting precipitate was dissolved in 50 mM sodium phosphate buffer containing 0.3 M sodium chloride, pH 7.0 (abbreviated as buffer C, hereinafter) and dialyzed against the same buffer. After stirring slowly overnight with Con A-Sepharose (40 ml) equilibrated with Buffer C, the Con A-Sepharose was filtered by glass-filter and filled up in a column (2.6 × 46 cm). The column was washed with three times volume of Buffer C and then eluted with buffer C containing $\alpha$ -methylmannoside to give fractions having enzymatic activity of NAG. The fractions were dialyzed against Buffer C. After the resultant was subjected to Protein G-Sepharose column (1.5 × 12 cm, Pharmacia) equilibrated with Buffer C, a passing fraction was loaded onto an antibody-column (2.8 × 3 cm) equilibrated with Buffer C, and then washed with Buffer C, followed by elution with 0.2 M glycine-HCl buffer, pH 3.0.

The eluate was immediately neutralized with 0.5 M sodium phosphate buffer, pH 7.5 and dialyzed against 0.01 M sodium phosphate buffer, pH 6.0 (abbreviated as Buffer D, hereinafter). The resultant was concentrated with Collodion Bag (Sartorius) and subjected to Mono Q column (0.5 × 5 cm) chromatography. A passing fraction containing NAG isozyme B which was not adsorbed to Mono Q column was collected, concentrated, and loaded onto Sephacryl S-200 column (2.6 × 73 cm) equilibrated with Buffer D containing 0.1 M sodium chloride. Fractions having enzymatic activity were collected to give a objective enzyme (NAG isozyme B) (c.a. 2.3 mg). NAG isozyme A separated by Mono Q column was also purified by Sephacryl S-200 to give 3.5 mg of that. The purity of these NAG isozymes was confirmed by separating these proteins by electrophoresis using SDS-PAGE gradient gel (Daiichi Pure Chemicals Co., Ltd.) and visualization with Coomassie brilliant blue.

As a result, under the non-reducing condition two bands of 56 K ($\alpha$ chain) and 60 K ($\beta$ chain) are appeared in case of NAG isozyme A and only one bands of 60 K ($\beta$ chain) in case of NAG isozyme B.

Under reducing condition, the band of 60 K is separated to two bands of 30 K ($\beta$ a chain) and 28 K ($\beta$ b chain) because $\beta$ chain of NAG isozyme A and B was made low molecule. The band of $\alpha$ chain of NAG isozyme A was not changed.

The purity of $\beta$ chain of NAG isozyme B under the non-reducing condition was examined by densitometer. As a result, NAG isozyme B was purified well.

(2) Preparation of enzyme-labeled antibody

Using horseradish peroxidase as an enzyme, an enzyme-labeled antibody was prepared by the hinge method.

To anti-NAG monoclonal antibody Hex 32 (18 mg) purified in above item 1. (1) (e) was added pepsin (878 $\mu$g/8.8 ml, Biehringer Mannheim) in 0.1 M citrate buffer, pH 4.1 and reacted at 37 °C for 18 hr to digest the antibody. The reaction mixture was separated by Superose 12 column (1.5 × 50 cm, Pharmacia). The obtained F(ab')$_2$ fraction was concentrated to 2.2 ml by Centricon 30 (Amicon) and reduced at 37 °C for 90 min with 0.1 M 2-mercaptoethylamine (1/10 volume) in 0.1 M phosphate buffer (pH 6.0) containing 5 mM ethylenediaminetetraacetic acid (EDTA). The resulting Fab' fraction was separated by Superose 12 column (1.5 × 50 cm) equilibrated with the same buffer and concentrated to 1 ml by Centricon 10.

To peroxidase (1.65 mg/2.47 ml, Sigma Chemical Co.) in 0.1 M phosphate buffer, pH 7.0 was added $\epsilon$-maleimidocaproyloxysuccinimide (EMCS) (13.1 mg/164 $\mu$l, Dojin) in N,N-dimethylformamide and reacted at 30 °C for 30 min. The reaction mixture was centrifuged to remove precipitate. The resulting supernatant was subjected to PD-10 column equilibrated with 0.1 M phosphate buffer (pH 6.0) to remove low molecule substances. Maleimidated-peroxidae thus obtained was reacted with Fab' fragment at 4 °C for 15 hr at a mole ratio of 1 : 1. The resulting peroxidase-labeled anti-NAG monoclonal antibody (Fab' fragment) was separated from reaction mixture by Superose 12 column (1.5 × 50 cm) equilibrated with 0.1 M phosphate buffer (pH 6.5) to yield 3.8 mg of the labeled antibody.

(3) Preparation of antibody-coated plate

Immunomodule Plate F-8H (Nunc) was coated with anti-NAG monoclonal antibody Hex 31 (10 $\mu$g/ml, 200 $\mu$l/well) in 10 mM PBS (pH 7.4) at 4 °C for 24 hr. The wells were washed three times with 10 mM PBS (pH 7.4) containing 0.05 % Tween 23, to which was added 10 mM PBS, pH 7.4 (300 $\mu$l/well) containing 10 % saccharose and 10 % BSA (Sigma Chemical Co.), followed by allowing to stand at room temperature for 1 hr. After removing the buffer from the wells, the plate was dried overnight under reduced pressure with silica gel in a desiccator to give an antibody-coated plate.

(4) Sandwich EIA

The antibody-coated plate prepared in above item (3) was washed once with 10 mM phosphate buffer, pH 7.4 (300 $\mu$l/well) containing 0.05 % Tween 20, 0.15 M sodium chloride, and 0.06 % Kathon CG (Rohm and Haas). To each well was added 150 $\mu$l of 0.4 M citrate buffer, pH 5.2 containing 0.1 % BSA and Kathon CG and 25 $\mu$l of standard antigen solution (0 - 80 ng/ml NAG isozyme B purified in above item 2. (1) in 10 mM phosphate buffer, pH 6.0 containing 0.1 % BSA, 0.1 % Kathon CG and 0.15 M sodium chloride) or urinary sample. After agitation, 1st reaction was carried out at 30 °C for 2 hr, followed by washing four times.

To each well was added 100 $\mu$l of labeled antibody solution (250 ng/ml labeled antibody prepared in above item 2. (2) in 10 mM phosphate buffer, pH 7.4 containing 0.1 % BSA, 0.1 % Kathon CG and 0.15 M sodium chloride), and 2nd reaction was carried out at 30 °C for 1 hr. After washing four times, 100 $\mu$l of substrate solution (4.3 mM 2,2'-azinobis ( 3-ethylbenzthiazoline-6-sulfate (ABTS) in 10 mM citrate buffer, pH 5.0 containing 2 mM hydrogen peroxide) was added to each well and enzyme reaction was carried out at 30 °C for 15 min. The enzyme reaction was stopped by adding 100 $\mu l$ of 10mM citrate buffer, pH 5.0 containing 0.05 % sodium azide. The absorbance was detected at 415 nm using microplate-reader (MTP-32, Corona Electric Co, Ltd.) and the amount of NAG isozyme B in urinary sample was determined from the standard curve.

(a) Standard curve and Cross-reactivity to NAG isozyme A

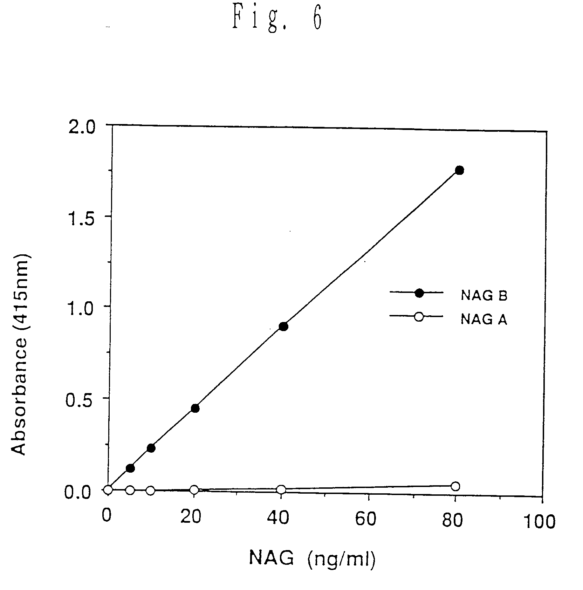

Standard curve of the present sandwich EIA using NAG isozyme B as a standard antigen was shown in Fig. 6 (-●-). It was excellently linear over the range from 0 to 80 ng/ml. The mean + 3SD as the lower limit of detection was 0.5 ng/ml (n = 20). Furthermore, this sandwich EIA was carried out using NAG isozyme A or bovine NAG as a standard antigen. As a result, the cross-reactivity to NAG isozyme A (-○-) amounted to 2 - 3 % and there is no cross-reactivity to bovine NAG.

(b) Reproducibility

The reproducibility of the present sandwich EIA was shown in Table 2. It was estimated using three urinary samples having different concentration. The within-assay C.V. was 2.5 - 5.4 % and the between-assay C.V. was 6.2 - 9.1 %.

(c) Dilution curve

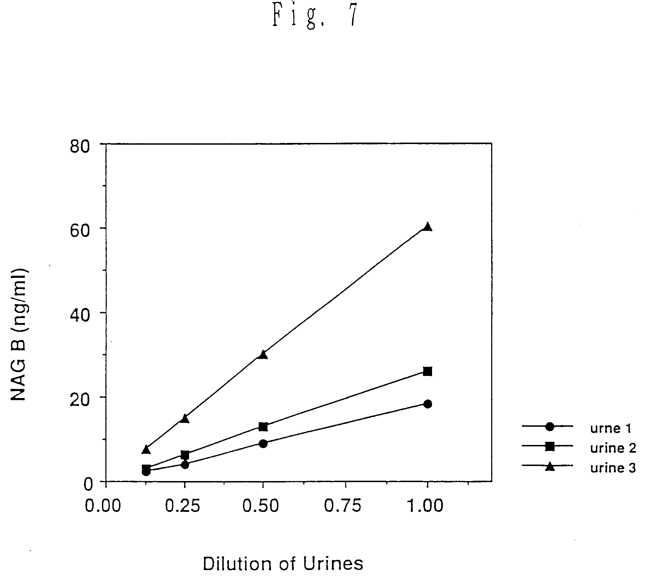

The dilution curves of three urinary samples having different concentration were shown in Fig. 7. They were excellently linear.

(d) Analytical recovery of standard added to urine

The recovery of standard antigen added to three urinary samples having different concentration ranged from 91 % to 114 % as shown in Table 3.

(e) Measurement of NAG isozyme B in urine of normal healthy human

NAG isozyme B in urine of normal healthy human was measured by age and sex. The means at early morning (n = 137, Table 4) and the other time (n = 177, Table 5) were 4.2 $\mu g$ and 3.3 $\mu g$ per 1 g of creatinine, respectively.

From these results, it was concluded that the present sandwich EIA made it possible to detect NAG isozyme B quantitatively and reproducibly. This sandwich EIA needed less amount of sample (25 $\mu l$), was simpler, and made it possible to treat the larger number of sample than the conventional NAG isozyme analysis such as chromatography or electrophoresis. In this sandwich EIA, furthermore, an edge effect, that is, a defect of an assay using a plate, was not observed. Therefore, the present sandwich EIA is adopted to routine clinical work.

# Table 1

| NAG(B) | 1 | 2 | 3 | 4 | 5 . | AVG | sp | SD | CV(%) | |
|---|---|---|---|---|---|---|---|---|---|---|
| 0 | 286 | 327 | 300 | 364 | 323 | 320 | 0 | 27 | 8.33 | |
| 0.5 | 915 | 945 | 932 | 878 | 920 | 918 | 598 | 23 | 2.45 | |
| 1 | 1371 | 1468 | 1411 | 1379 | 1434 | 1413 | 1093 | 36 | 2.53 | |
| 2 | 2384 | 2299 | 2254 | 2660 | 2660 | 2451 | 2131 | 175 | 7.15 | |
| 5 | 4773 | 5573 | 5344 | 6195 | 6065 | 5590 | 5270 | 514 | 9.19 | |
| 10 | 10616 | 10916 | 10249 | 10261 | 10641 | 10537 | 10217 | 253 | 2.40 | |
| | | | | | | | | | | |
| urine | 1 | 2 | 3 | 4 | 5 | AVG | sp | SD | CV(%) | RIA |
| 8 | 1135 | 1196 | 1227 | 1237 | 1209 | 1201 | 881 | 36 | 2.98 | 0.79 |
| 4 | 1796 | 1548 | 1790 | 1642 | 1714 | 1698 | 1378 | 94 | 5.52 | 1.27 |
| 11 | 1588 | 1626 | 1672 | 1625 | 1549 | 1612 | 1292 | 41 | 2.56 | 1.19 |
| 7 | 2196 | 1991 | 2169 | 2062 | 2051 | 2094 | 1774 | 77 | 3.67 | 1.66 |
| 27 | 3793 | 3653 | 4096 | 3662 | 3657 | 3772 | 3452 | 170 | 4.51 | 2.84 |
| 26 | 2291 | 2320 | 2457 | 2213 | 2159 | 2288 | 1968 | 102 | 4.45 | 1.84 |
| 64 | 4914 | 5245 | 5255 | 5235 | 4949 | 5120 | 4800 | 154 | 3.01 | .3.70 |
| 29 | 4052 | 4300 | 4208 | 4467 | 4284 | 4262 | 3942 | 135 | 3.16 | 3.15 |

Table 2

| Table 2. Precision of The Present EIA | | | | |
|---|---|---|---|---|
| Assay | n | Mean,ng/ml | SD,ng/ml | CV,% |
| Within-assay | | | | |
| urine 1 | 8 | 5.7 | 0.3 | 5.4 |
| urine 2 | 8 | 20.0 | 0.5 | 2.5 |
| urine 3 | 8 | 37.1 | 1.8 | 4.8 |
| Between-assay[a] | | | | |
| urine 1 | 14 | 6.4 | 0.6 | 9.1 |
| urine 2 | 14 | 19.8 | 1.2 | 6.2 |
| urine 3 | 14 | 35.2 | 2.3 | 6.6 |

[a]Fourteen separate assayson 14 days (duplicate of each samples).

# Table 3

| Table 3.  Analytical Recovery of Standard Added to Urines | | | |
|---|---|---|---|
| Urine No. | Endogenious NAG Isozyme B | Added | Found[a] Recovery |
| | ng/ml | ng/ml | ng/ml   % |
| 1 | 8.9 | 5.0 | 5.5   110 |
| | 8.9 | 12.5 | 12.9   103 |
| | 8.9 | 25.0 | 23.9   96 |
| 2 | 13.1 | 12.5 | 11.4   91 |
| | 13.1 | 25.0 | 25.4   102 |
| | 13.1 | 50.0 | 47.9   96 |
| 3 | 27.3 | 12.5 | 14.2   114 |
| | 27.3 | 25.0 | 28.1   112 |
| | 27.3 | 50.0 | 46.1   92 |

[a] Increase in NAG isozyme B over endogeneous

EP 0 575 906 A2

Table 4

| Age | Sex | Number of individuals | NAG-B activity | |
|---|---|---|---|---|
| | | | ng/ml | $\mu$g/g Cr. |
| 20-29 | male | 3 | 5.6±2.6 | 1.9±0.9 |
| | female | 4 | 8.6±6.0 | 3.8±2.0 |
| 30-39 | male | 20 | 4.2±3.5 | 3.2±4.2 |
| | female | 6 | 6.0±1.4 | 4.3±1.0 |
| 40-49 | male | 20 | 7.7±6.1 | 4.6±3.5 |
| | female | 11 | 4.9±2.5 | 6.3±3.9 |
| 50-59 | male | 20 | 5.4±5.5 | 4.1±3.1 |
| | female | 20 | 4.7±2.1 | 3.8±2.7 |
| 60-69 | male | 13 | 6.6±3.9 | 6.1±5.0 |
| | female | 20 | 3.9±1.8 | 3.4±1.5 |
| | male | 76 | 5.9±5.0 | 4.2±3.9 |
| | female | 61 | 4.1±2.9 | 4.2±2.7 |
| total | | 137 | 5.1±4.3 | 4.2±3.4 |

Table 5

| Age | Sex | Number of individuals | NAG-B activity | |
|---|---|---|---|---|
| | | | ng/ml | $\mu$g/g Cr. |
| 20-29 | male | 20 | 3.2±2.4 | 1.9±0.9 |
| | female | 16 | 4.3±3.6 | 3.2±1.3 |
| 30-39 | male | 20 | 3.3±1.9 | 2.1±1.0 |
| | female | 19 | 2.8±2.2 | 2.7±1.8 |
| 40-49 | male | 21 | 2.5±1.6 | 2.6±1.6 |
| | female | 20 | 3.2±2.8 | 3.3±2.5 |
| 50-59 | male | 19 | 3.8±3.0 | 4.2±1.7 |
| | female | 20 | 2.8±2.3 | 4.1±2.1 |
| 60-69 | male | 9 | 4.6±4.0 | 5.8±3.5 |
| | female | 13 | 3.3±2.0 | 4.7±2.0 |
| | male | 89 | 3.3±2.5 | 3.0±2.1 |
| | female | 88 | 3.2±2.6 | 3.5±2.1 |
| total | | 177 | 3.3±2.6 | 3.3±2.1 |

## Claims

1. A sandwich immunoassay for $\beta$-N-acetylglucosaminidase (NAG), which comprises sandwiching NAG between an immobilized anti-NAG monoclonal antibody and a labeled anti-NAG monoclonal antibody.

2. A sandwich immunoassay for NAG, which comprises the following steps:
(1) reacting an immobilized anti-NAG monoclonal antibody and a labeled anti-NAG monoclonal antibody with a sample containing NAG to form a complex of immobilized antibody-NAG-labeled antibody and
(2) detecting the radioactivity or enzymatic activity of said labeled monoclonal antibody which is contained or is not contained in the complex.

14

3. A sandwich immunoassay for NAG of Claim 2, which further comprises the following steps:

(1) immobilizing an anti-NAG monoclonal antibody on a carrier to give an immobilized monoclonal antibody and

(2) labeling an anti-NAG monoclonal antibody which recognizes a different site from that recognized by the immobilized monoclonal antibody with a radioisotope or an enzyme to give a labeled monoclonal antibody.

4. The sandwich immunoassay of Claim 2 or 3, wherein the reaction of said immobilized monoclonal antibody and said labeled monoclonal antibody with a sample containing NAG is carried out simultaneously or separately.

5. The sandwich immunoassay of Claim 1, 2, 3, or 4, wherein said NAG is NAG isozyme B.

6. The sandwich immunoassay of Claim 1, 2, 3, or 4, wherein said NAG is NAG isozymes B and I.

7. The sandwich immunoassay of Claim 1, 2, 3, 4, 5, or 6, wherein said immobilized monoclonal antibody and said labeled monoclonal antibody are obtainable from monoclonal antibody Hex 31 and monoclonal antibody Hex 32 which are produced by Hybridoma Hex 31 (FERM BP-3858) and Hybridoma Hex 32 (FERM BP-3859), respectively.

8. The sandwich immunoassay of Claim 7, wherein said immobilized monoclonal antibody is obtained by immobilizing monoclonal antibody Hex 31 which is produced by Hybridoma Hex31 (FERM BP-3858).

9. The sandwich immunoassay of Claim 7, wherein said labeled monoclonal antibody is obtained by labeling monoclonal antibody Hex 32 which is produced by Hybridoma Hex32 (FERM BP-3859).

10. A monoclonal antibody recognizing NAG, which is useful for the sandwich immunoassay of Claim 1, 2, 3, 4, 5, or 6.

11. The monoclonal antibody of Claim 10, wherein said NAG is NAG isozyme B.

12. The monoclonal antibody of Claim 10, wherein said NAG is NAG isozymes B and I.

13. The monoclonal antibody of Claim 10, 11, or 12, which does not inhibit enzymatic activity of NAG.

14. The monoclonal antibody of Claim 10, which is monoclonal antibody Hex 31 produced by Hybridoma Hex 31 (FERM BP-3858) or monoclonal antibody Hex 32 produced by Hybridoma Hex 32 (FERM BP-3859).

15. A kit for the measurement of NAG, which comprises an immobilized anti-NAG monoclonal antibody and a labeled anti-NAG monoclonal antibody, wherein NAG can be sandwiched between said immobilized monoclonal antibody and said labeled monoclonal antibody.

16. The kit of Claim 15, wherein said NAG is NAG isozyme B.

17. The kit of Claim 15, wherein said NAG is NAG isozymes B and I.

18. The kit of Claim 15, 16, or 17, wherein said immobilized monoclonal antibody and said labeled monoclonal antibody are obtainable from monoclonal antibody Hex 31 and monoclonal antibody Hex 32 which are produced by Hybridoma Hex 31 (FERM BP-3858) and Hybridoma Hex 32 (FERM BP-3859), respectively.

19. The kit of Claim 18, wherein said immobilized monoclonal antibody is obtained by immobilizing monoclonal antibody Hex 31 which is produced by Hybridoma Hex31 (FERM BP-3858).

20. The kit of Claim 18, wherein said labeled monoclonal antibody is obtained by labeling monoclonal antibody Hex 32 which is produced by Hybridoma Hex 32 (FERM BP-3859).

# Fig. 1

## (A)

## (B)

# Fig. 2

(A)

Hex 31

(B)

Hex 32

# Fig. 3

Count (cpm)                                    NAG(B) standard

# Fig. 4

(A)

(B)

# Fig. 5

(A)

MonoQ HR5/5     urine 65

(B)

MonQ HR5/5     urine 86

Fig. 6

Fig. 7